# EUROPEAN PATENT APPLICATION

(11) **EP 1 792 660 A1**
(43) Date of publication of application: **06.06.2007**
(21) Application number: 06000276.3
(22) Date of filing: 09.01.2006
(51) Int. Cl.: B05B 1/04, B05B 1/26, A61M 15/00

(54) **Dispensing device**

(30) Priority: 02.12.2005 EP 05026285
(71) Applicant: Boehringer Ingelheim Pharma GmbH & Co. KG, 55216 Ingelheim am Rhein (DE)
(72) Inventor: Rohrschneider, Marc, 58093 Hagen (DE); Vehdelmann, Matthias, 59846 Sundern (DE); Dunne, Stephen Terence, Dr., Stowmarket Suffolk, IP14 3AE (GB)
(74) Representative: Gesthuysen, von Rohr & Eggert

(57) **Abstract**

A dispensing device (1) is proposed for dispensing a liquid (2), in particular containing or consisting of a drug, as a fine spray (3). A high fraction of small particles can be achieved by using a duct (5) or slit (22) having a flat cross section.

## Description

The present invention relates to a dispensing device for dispensing a liquid, in particular containing or consisting of a drug, as a fine spray. Preferably, the present invention relates to such an inhaler.

Drugs delivered through dispensing devices, in particular inhalers, are intended to optimally target specific sites in the pulmonary system. These sites include the nasal passages, the throat, and various locations within the lungs, such as the bronchi, bronchioles and alveolar regions. The ability to deliver drugs to a target area depends inter alia on the aerodynamic sizes of the particles or droplets. As currently believed to be understood, particles having an aerodynamic diameter of less than 2 µm are considered to be potentially optimal for deposition in the alveolar region of the lung. Particles that have an aerodynamic diameter of between 2 and approximately 5 µm may be more suitable for delivery to the bronchiole or bronchi regions. Particles with an aerodynamic size range greater than 6 µm, and more preferably 10 µm, are typically suitable for delivery to the laryngeal region, throat or nasal passages.

In most cases, it is desired to achieve a high inhalable fraction and a high delivery efficiency, i.e. the fraction that reaches the desired region, in particular in the lung. This depends on various factors, in particular on the characteristics of the generated spray plume, such as propagation velocity of the plume, particle size and its distribution, fraction of small particles, fraction of gas and the like. In the present invention, the desired spray plume characteristics include preferably a small particle size, a high fraction of drug particles with a diameter of 6 µm or less, a low propagation velocity and/or a long duration of spray generation and possible inhalation. Thus, a so-called soft mist can be achieved.

In particular, the present invention is concerned with so-called metered dose inhalers (MDIs), which should be named more correctly metering dose inhalers, for the delivery of drugs to the lungs. Liquid containing a drug is in a carnister under gas pressure and can be released via a metering valve. More generally, the present invention relates to inhalers for dispensing a liquid containing or consisting of a drug. In the present invention the term "liquid" has to be understood preferably in a broad sense covering inter alia solutions, suspensions, dispersions or the like.

Many MDIs are on the market. There are two main distinct types, namely the suspension type and the solution type. In the suspension type, the drug is in solid powder form suspended in at least one liquefied gas and/or at least one other liquid In the solution type, the drug is dissolved in at least one liquefied gas and/or at least one other liquid. It is also possible to use a drug in form of a so-called suslution (mixture of suspension and solution). The present invention may relate to all these types of MDIs.

All MDIs suffer from two main problems. Firstly, the delivery time of the drug is extremely short and makes patient coordination difficult. Secondly, the exit velocity of the drug is high and leads to substantial deposition of the drug in the mouth and throat, i.e. the delivery efficiency is low.

WO 92/12799 A1 discloses a pre-metered dispensing device for transforming a flow of fluid into a spray of fine particle size, wherein an annular flow through a duct is caused with a velocity gradient within that flow sufficient to cause sheer forces between components of the flow to break the flow up into a spray. The cross sectional shape of the duct is preferably circular, but other cross sectional shapes, for example an irregular cross section or a polygonal cross section can be used. However, the known device and method are not optimal for generating a slow spray plume with the desired characteristics.

WO 2004/041326 A2 discloses a tubular nozzle for use in MDIs. The tubular nozzle is curvilinear throughout a defined length and has a curved portion with a radius of curvature of at least 2.5 times the inner diameter of the tubular nozzle. The cross section of the tubular nozzle may be selected from a wide range of choices such as circular, oval, square, rectangular, polygonal, and the like. Measurements have shown that this design is not suitable for achieving the desired spray plume characteristics, in particular a high fraction of fine particles.

Object of the present invention is to provide an improved dispensing device, wherein the desired spray plume characteristics can be realized, in particular a high fraction of fine particles.

The above object is achieved by a dispensing device according to claim 1 or 6. Preferred embodiments are subject of the subclaims.

One aspect of the present invention is to provide a nozzle duct with a flat cross section. The liquid is forced through the duct by gas pressure to generate a spray including fine particles. The ratio of the largest side to the smallest side of the flat cross section of the duct is at least 2.0. Surprisingly, significantly higher delivery efficiency can be achieved than by a circular or quasi circular duct. This effect may be explained in that the flat cross section provides a larger perimeter for a given cross sectional area than a non-flat cross section. This larger perimeter results in a larger duct surface that is in contact with the drug or liquid so that better breaking up can be achieved due to higher sheer forces preferably without changing the cross sectional area (hydraulic diameter), i.e. without changing the mass flow significantly.

Preferably, the ratio of the largest side to the smallest side of the flat cross section is between 2 and 20, most preferably about 3 to 10. Thus, a high output of liquid with small particle size can be achieved. The dispensing device produces a spray plume with a high inhalable fraction (fine particles with diameter of 6 µm or less) and with the desired spray plume characteristics.

The dispensing device with the proposed nozzle design may substantially increase the drug delivery time and slow the exit velocity without adversely affecting the mean particle size of the spray. Alternatively or additionally, a high fraction of small particles may be achieved. Thus, the spray may exit as a soft mist instead of a fast moving plume.

According to an alternative or additional aspect of the present invention, the nozzle comprises a nozzle or duct with at least one sharp change in cross section and/or with alternate sections having large and small cross sectional areas. Surprisingly, this improves atomization, in particular formation of a high fraction of small particles, as well.

Preferably, the duct is a capillary tube or the like.

The duct may have one or more bends.

The total cross sectional area of the duct is preferably between 0.02 and 0.4 mm².

The duct preferably has a hydraulic diameter of between 20 and 1000 µm.

More than one duct may be used in parallel.

The duct preferably has a length of between 5 and 1000 or more, in particular between 10 and 250, hydraulic diameters (the hydraulic diameter is defined as the ratio of 4 cross sectional areas over the duct perimeter or for a rectangular duct of sides x and y as 4xy/[2(x+y)]).

For any given pressure the longer the duct the slower is the delivery to the patient.

The duct can be made of any material that is drug compatible including plastics, ceramics or metals. The duct is preferably formed by molding a groove in a plastic body and sealing this with a second plastic part or any other suitable part to form the duct.

The duct may be modified to decrease the exit velocity of the spray, and, hence, increase the inhalable fraction. In one embodiment, the duct ends with a diffuser to deaccelerate the flow. The total angle of the diffuser is preferably less than 10 degrees. In another embodiment, the flow is deaccelerated by causing the exit flows of more than one duct to collide at an angle deaccelerating the flow.

Alternatively, the nozzle may comprise a slit with a dimension, in particular a capillary dimension, similar to the flat duct. The slit may lead to similar effects as the flat duct. In particular, it has been found that the slit has the greatest effect of incrasing the fine fraction if placed at the exit of a flat duct. The slit may be rectangular, half circle or crescent in cross sectional area.

The nozzle may be used with both suspension and solution type MDIs or other inhalers.

The dispensing device is preferably gas powered. Any gas may be used. For instance liquefied gases such as HFA134a and HFA227, or compressed gases may be used. In particular, the gas is stored in a canister or reservoir together with the drug / liquid and released preferably via a metering valve.

Further aspects, advantages and features of the present invention will be apparent from the claims and the following detailed description of preferred embodiments. In the drawings show:
- Fig. 1: a schematic partial sectional view of a dispensing device with a nozzle having a duct according to one embodiment of the present invention;
- Fig. 2: a schematic partial sectional view of the dispensing device according to Fig. 1 during dispensing;
- Fig. 3: a cross sectional view of the duct with a first cross section;
- Fig. 4: a cross sectional view of the duct with a second cross section;
- Fig. 5: a cross sectional view of the duct with a third cross section;
- Fig. 6: a schematic longitudinal sectional view of the duct according to another embodiment;
- Fig. 7: a first cross sectional view of the duct according to Fig. 6;
- Fig. 8: a second cross sectional view of the duct according to Fig. 6;
- Fig. 9: a schematic longitudinal view of the duct with a diffuser;
- Fig. 10: a schematic sectional view of the duct with a tapered inlet section;
- Fig. 11: a schematic sectional view of a duct with a multiple jet impinging means;
- Fig. 12: a schematic sectional view of two ducts forming a multiple jet impinging means;
- Fig. 13: a schematic partial sectional view of a dispensing device with a nozzle having two ducts according to another embodiment of the present invention;
- Fig. 14: a partial sectional view of a nozzle with a slit member according to a further embodiment of the present invention; and
- Fig. 15: a top view of the slit member.

In the Fig., the same reference signs are used for same or similar components, wherein same or similar characteristics, features or advantages are or can be realized or achieved even if a repeated discussion is omitted. Further, the features and aspects of the different embodiments can be combined in any desired manner and/or used for other dispensing devices or inhalers for dispensing liquid.

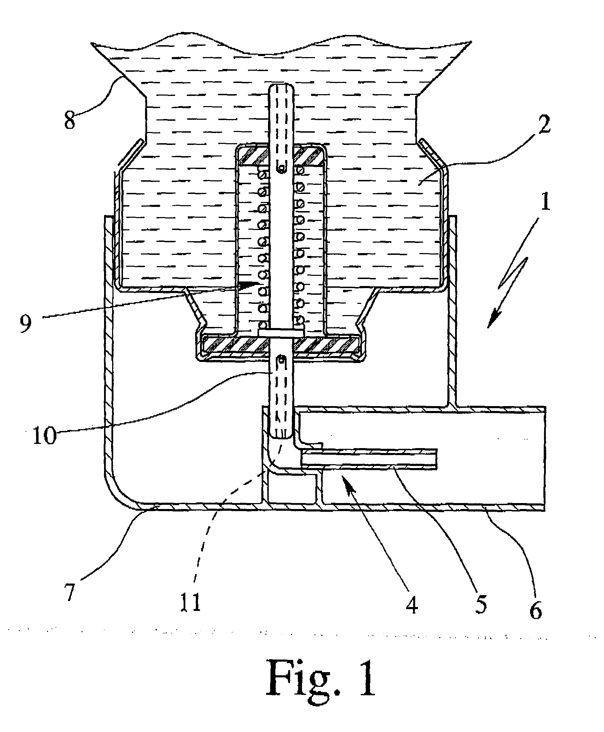

Fig. 1 shows in a schematic partial sectional view - for illustration purposes not in scale - a dispensing device 1 according to the present invention. The dispensing device 1 is preferably gas powered. Preferably, the dispensing device 1 is a preferably oral or nasal inhaler, in particular a MDI for a user or patient (not shown).

The dispensing device 1 is designed to dispense liquid 2 which in particular contains or consists of at least one drug. The liquid 2 may be or contain a pure drug or a mixture of at least two drugs. Additionally or alternatively, the liquid 2 may contain at least one other substance, such as a solvent or liquefied gas. In particular, the drug itself may be liquid or solid

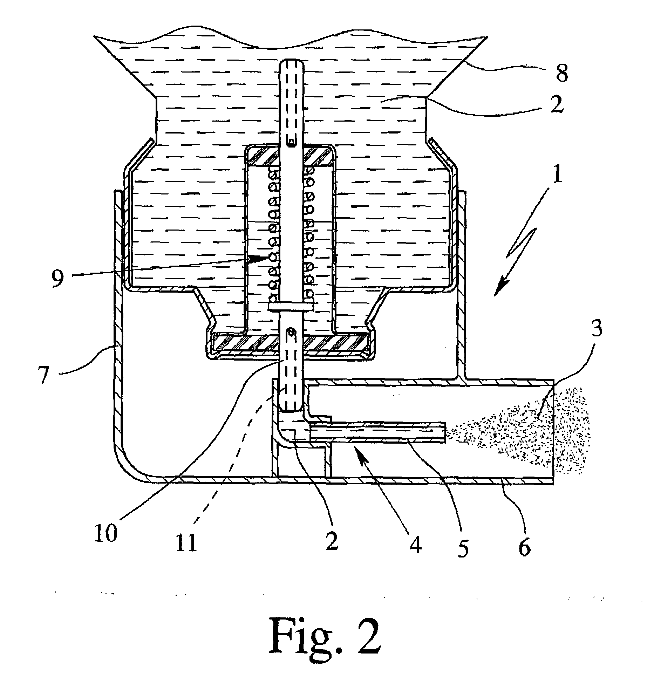

Fig. 2 shows the dispensing device 1 when dispensing the liquid 2 as a spray 3 in a very schematic manner similar to Fig. 1. The spray 3 comprises fine, preferably liquid particles, i.e. has fine particle size of preferably 6 µm or less. In particular, the spray 3 has the desired spray plume characteristics as described above.

The dispensing device 1 comprises a nozzle 4 having a duct 5 through which the liquid 2 can be dispensed for forming the spray 3.

The nozzle 4 with its duct 5 is preferably located in or integrated into a mouthpiece 6 for oral inhalation and/or a spray head 7 of a dispensing device 1. Preferably, the outlet of the nozzle 4 / duct 5 is retracted within the mouthpiece 6. This facilitates forming of a slow spray plume within the mouthpiece 6 that can be inhaled by a user or patient (not shown). However, other constructional solutions are possible as well.

The dispensing device 1 is adapted to receive or comprises a reservoir 8 for storing the liquid 2. The reservoir 8 may be integrated into the dispensing device 1 or form part of the dispensing device 1. Alternatively, the reservoir 8 may be a separate part, in particular a bottle, can or container, that can be inserted or connected with the dispensing device 1 and optionally replaced.

The dispensing device 1 uses gas pressure to force the liquid 2 through the nozzle 4 / duct 5 to generate the spray 3 with fine particle size. In particular, compressed or more preferred liquefied gas is used. This gas is preferably contained in the reservoir 8 / liquid 2. Consequently, the reservoir 8 is under pressure. Depending on the filling level, a gas space (not shown) will form within the reservoir 8. However, other means for pressurizing the liquid 2 could be used as well.

The dispensing device 1 preferably comprises a regulation or control means, in particular a metering valve 9 as shown in Fig. 1 and 2. Fig. 1 shows the metering valve 9 in its closed position where no liquid 2 is dispensed. Fig. 2 shows the metering valve 9 in its open position, i.e. where liquid 2 is dispensed as spray 3.

Any suitable metering valve 9 or any other regulation or control means can be used. In the present embodiment, the metering valve 9 comprises a stem 10 with a preferably axial outlet channel 11.

The nozzle 4 / duct 5 is fluidically connected to the metering valve 9, in particular its stem 10 / outlet channel 11. In the shown embodiment, the flow path from the outlet channel 11 to the duct 5 comprises at least one bend and may be formed by the head 7, by the nozzle 4 or by any other connecting element. However, other constructional solutions are possible as well. For example, the nozzle 4 / duct 5 could be directly formed by the valve 9 or its stem 10.

For dispensing a dose of drug / liquid 2, the valve 9 is actuated, preferably by depressing the stem 10, head 7 or any other suitable actuation member. Alternatively, an electric valve or the like could be used and e.g. electrically opened.

Then, the gas pressure forces a dose of drug / liquid 2 out of valve 9 / channel 11. During this dispensing operation, the gas significantly expands so that the mixture of liquid and/or solid particles and gas has a high volume fraction of gas, in particular of more than 1000 or 10000 at ambient pressure, and is forced through the nozzle 4, i.e. in the first embodiment through duct 5. Thus, the spray 3 is generated.

When releasing the head 7 or any other actuation member, the valve 9 returns to its closed state shown in Fig. 1 and refills with the next dose of liquid 2 for the next dispensing operation.

In one embodiment, the valve 9 or its outlet channel 11 comprises a smaller cross sectional area than the duct 5 so that the gas flow is determined at least mainly by the duct 5 during dispensing. This is favourable for forming a slow spray plume, i.e. a soft mist.

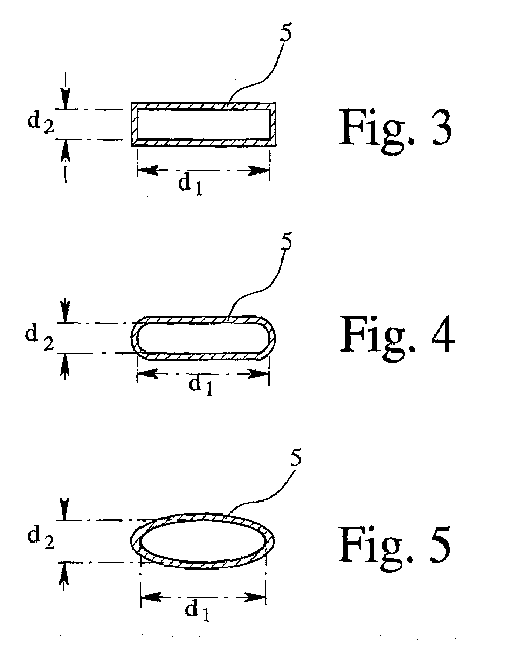

According to one aspect of the invention, the duct 5 has a flat (inner) cross section. Fig. 3 to 5 show potential cross sections of the duct 5. Fig. 3 shows a substantially rectangular cross section. Fig. 4 shows a flat cross section with two opposite straight sides connected by two curved portions. Fig. 5 shows an oval or elliptical cross section.

In the present invention, a cross section is considered to be flat when the ratio of the largest side d1 to the smallest side d2 of the cross section is at least 2.0. Preferably, the ratio is between 2 to 20 and in particular about 3 to 10. It is pointed out that the cross sections shown in Fig. 3 to 5 are not in scale.

The largest side d1 is preferably between 0.3 to 5 mm, in particular 0.5 to 1 mm. Most preferably, the ratio of the largest side d1 to the (desired) fine particle size (mass mean diameter of the particles of the spray 3 of about 2 to 6 µm) is less than 500, preferably less than 300, in particular about 30 to 300.

The smallest side d2 is preferably between 0.05 to 0.3 mm, in particular about 0.07 to 0.2 mm. Most preferably, the ratio of the smallest side d2 to the mass mean (desired) fine particle size (mass mean diameter of the particles of the spray 3 of about 2 to 6 µm) is less than 50, preferably less than 30, in particular about 10 to 20.

The length of the duct 5 means the length with the flat cross section. Thus, the duct 5 can have a larger length, i.e. further portions with another cross sectional shape and/or with a larger cross sectional area so that the influence of these other portions is low on the spray generation in comparison to the portion of the duct 5 with the flat cross section. However, the cross sectional area and/or the shape of the flat cross section may vary over the length of the duct 5 (the portion with the flat cross section). Thus, it is possible that the cross sectional area of the duct 5 tapers from the inlet to the outlet or vice versa.

Most preferably, the duct 5 comprises at least one portion of flat cross section with constant cross sectional area, i.e. constant diameter and/or shape.

The length of the duct 5 - i.e. the portion with flat cross section - may be in the range of 3 mm to 80 mm, in particular 5 to 30 mm. Preferably, the duct length is adapted to the mean hydraulic diameter of the duct 5 such that the ratio of the length of the duct 5 to the mean hydraulic diameter is at least 5, in particular about 10, or more.

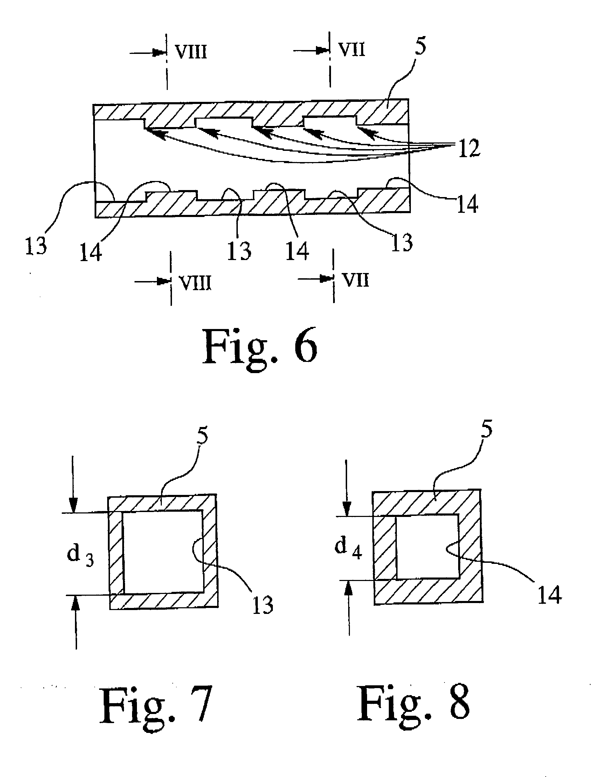

Fig. 6 shows in a schematic longitudinal sectional view another embodiment of the nozzle 4 with a duct 5. In this embodiment, the duct 5 comprises at least one sharp change 12 in cross section. In this embodiment, multiple sharp changes 12 are provided in sequence. Alternatively or additionally, the duct 5 comprises alternate sections 13 and 14 with large and small sectional areas. Preferably, the transitions between the alternate sections 13, 14 are sharp or step-like and form the changes 12. in particular, these transitions are formed by inner surface areas of the nozzle 4 or duct 5 that extend substantially radially or transversally to the main flow direction or to the longitudinal axis of the duct 5.

In the shown embodiment, the cross sectional shape may be essentially square. However, the cross section shape may also be substantially flat, oval or rectangular in particular or as shown in Fig. 3 to 5.

Fig. 7 shows a cross sectional view of the duct 5 according to line VII-VII in Fig. 6, i.e. of a section 13. Fig. 8 shows a similar cross sectional view of the duct 5 according to line VIII-VIII of Fig. 6, i.e. of a section 14.

The ratio of the side d3 of the large section 13 to the side d4 of the small section 14 is preferably 1.1 or more. In particular, the change 12 is more than 20%, preferably more than 50%, in cross sectional area.

Preferably, the alternate sections 13, 14 have similar cross sectional shapes.

The ratio of the cross sectional areas of the large sections 13 to the cross sectional areas of the small sections 14 is preferably more than 1.2, in particular more than 1.5 up to about 3.

Studies have shown surprisingly clearly that the embodiment according to Fig. 6 leads to a significantly higher fraction of small particles and/or to a significant decrease of the exit velocity of the spray 3 in comparison to a continoues duct 5. Consequently, the embodiment supports the achievement of desired spray plume characteristics. Further, this embodiment can be combined with the flat cross section of the duct 5.

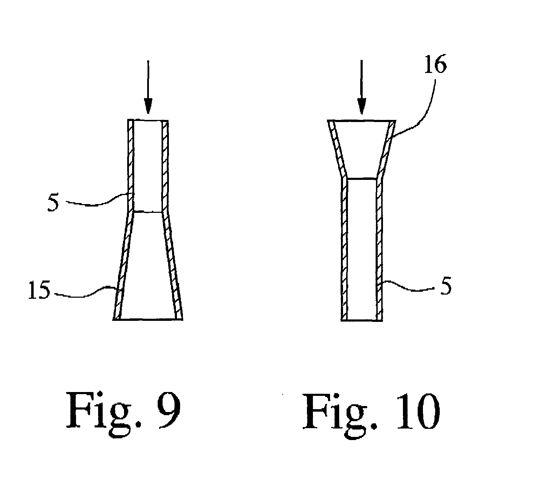

Fig. 9 shows a longitudinal sectional view of another embodiment of the duct 5. Here, the dispensing device 1 or in particular the duct 5 comprises a means for slowing down the outlet velocity and, thus, the propagation velocity of the spray 3. In this embodiment, the means for slowing down the velocity is a diffuser 15 located at or connected to the exit of the duct 5. The arrow shows the direction of flow. The diffuser 15 has an appropriate angle, preferably less than 10 degrees to the longitudinal axis, to decrease the outlet velocity.

Additionally or alternatively, the duct 5 can also comprise a tapered inlet section 16 as shown in Fig. 10 in a longitudinal section view similar to Fig. 9. The arrow shows the direction of flow. The tapered cross section 16 can have any suitable inner contour and may be curved to avoid any sharp edges at the transition to the tapered section 16 or from the tapered section 16 to the duct 5. This tapered inlet section 16 may be used for all embodiments with a duct 5 or multiple ducts 5. This applies also for the embodiment according to Fig. 9, i.e. the means for slowing down the outlet velocity.

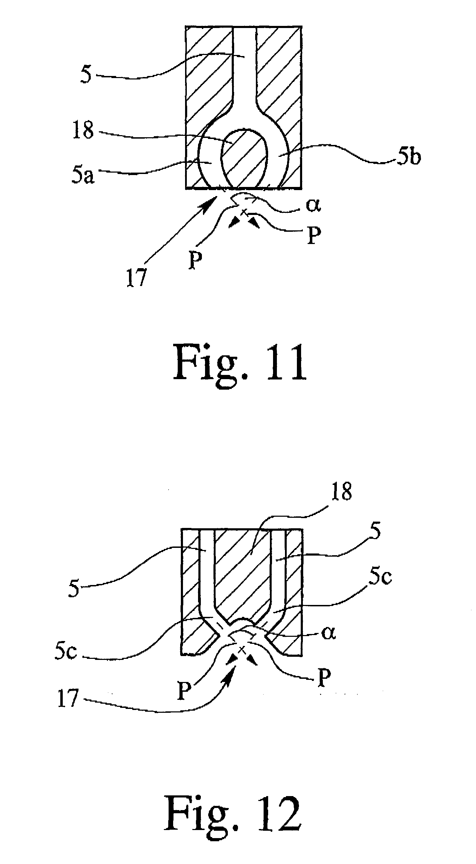

Fig. 11 shows in a schematic sectional view another duct arrangement with another means for slowing down the velocity which forms a multiple jet (spray) impinging means 17. The means 17 forms multiple - at least two - jets P which impinge, i.e. hit each other as indicated in Fig. 11. In this embodiment, the duct 5 divides into two sections 5a and 5b that are designed such that the openings or outlets are inclined to each other so that the jets P ejecting from the portions 5a and 5b are inclined to each other and impinge. For example, a flow divider 18 or any guiding means can be located in the flow path to form the at least two sections 5a and 5b of the duct 5 as shown in Fig. 11.

The impinging angle α between the jets P is between 30° and 180°, preferably about 90°. The impinging of the jets P results in a decrease of the velocity of the spray 3 and/or in a further breaking up of particles and/or in better focusing of the spray 3. These effects depend on the impinging angle α. These angles also apply for the following embodiment.

Fig. 12 shows in a schematic sectional view another embodiment of the jet impinging means 17. Here, two or more ducts 5 comprise inclined or outlet sections 5c which are inclined to each other so that the jets P ejected from outlet sections 5c impinge with each other.

The embodiments according to Fig. 11 to 12 are also suitable for impinging more than two jets P. For example, it is possible to have similar arrangements in the cross sectional planes perpendicular to the drawing plane resulting in four outlet directions and jets P arranged on the surface of a conus. However, multiple other arrangements with similar effects are possible.

It has to be added that the cross sections of the duct sections 5a to 5c can be flat, but can have any other suitable cross sectional shape.

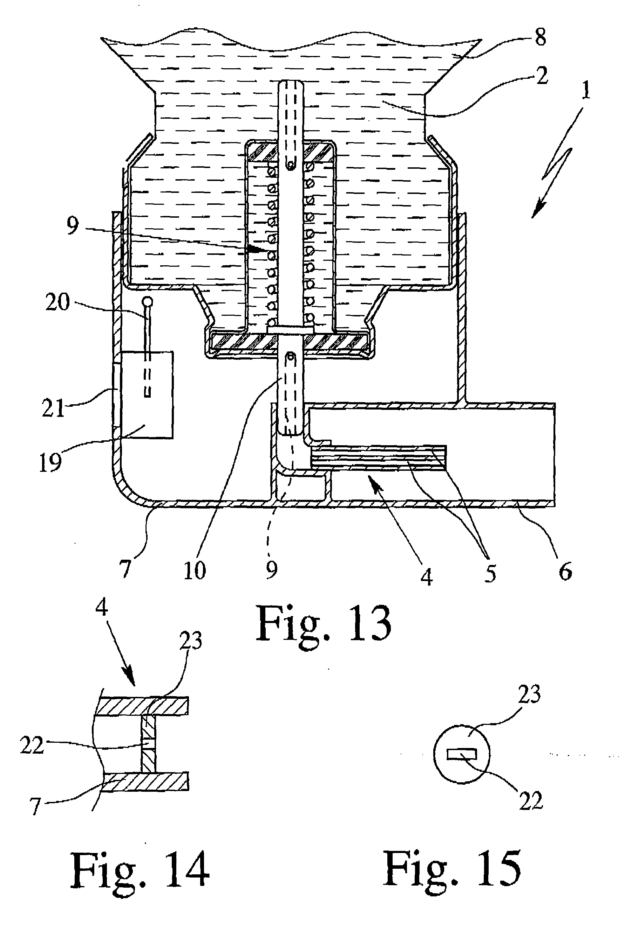

Fig. 13 shows in a schematic partial sectional view the dispensing device 1 according to another embodiment of the present invention. In this embodiment, the nozzle 4 has multiple ducts 5, in particular two or more ducts 5 in parallel. The ducts 5 can dispense simultaneously one dose of the drug / liquid 2, in particular for increasing the total mass flow or output so that a desired dose can be discharged or dispensed in a sufficiently short time as desired and/or required.

Regarding the structure, dimensions, features and the like of the dispensing device 1 and of the duct 5 reference is made to the above explanations with regard to the other embodiments. These explanations apply as well.

The dispensing device 1 may comprise a monitoring device 19, in particular for counting the number of previous dispensing operations and/or of the remaining dispensing operations / doses available for a user: The monitoring device 19 may comprise a sensor element 20, e.g. a stylus, a micro switch, a pressure sensor, a flow sensor or the like, for detecting actuations or any actual dispensing or the generation of the spray 3. The monitoring device 19 may work mechanically and/or electrically / electronically. Preferably, the monitoring device 19 comprises a display 21 for displaying the current count number and/or any additional user information, e.g. kind of drug, date / time of first or last use and the like.

In the shown embodiment, the monitoring device 7 is integrated into the dispensing device 1, in particular into the head 7. However, the monitoring device 19 can be arranged at other locations and/or separately from head 7.

Fig. 14 shows in a partial sectional view the nozzle 4 according to another embodiment. The nozzle 4 may be formed by the head 7 or any other guiding means and comprises a slid 22 in a slit member 23.

Preferably, the slit 22 has similar dimensions as the flat duct 5 described above, i.e. the slit 22 is flat or small. The dimensions of the slit 22 preferably correspond to the preferred cross section of the flat duct 5 as described above.

Fig. 15 shows in a top view the slit member 23 which may have a disc-like shape as shown. However, any other suitable shape is possible.

Preferably, the mean duration of the spray 3 is at least 0.2 or 3 s, in particular about 0.5 to 2.5 s.

It has to be noted, that the dispensing device 1 can be used for dispensing one drug, a blend of drugs or at least two or three separate drugs. In the latter case, the separate drugs or liquids 2 are stored in separate storage chambers or reservoirs 4 and, during the dispensing operation, the drugs / liquids 2 are mixed and discharged through a common duct 5 or through separate ducts 5. It is also possible to mix the separate drugs / liquids 2 by impinging jets P of the separate drugs.

According to a further embodiment, the dispensing device 1 may be breath activated, in particular wherein the formulation 2 is only released after the patient's or user's inhalation rate has reached a predetermined level, preferably by the use of a pressure sensitive means, such as a bursting element, membrane or valve, or any other mechanism.

According to another aspect, the present invention is used in or for a dispensing device / inhaler that is offered under the trademark "RESPIMAT" by Boehringer Ingelheim KG and/or that is constructed or designed according to WO 91/14468 A1, WO 97/12687 A1 (in partiuclar Fig. 6a, 6b) and/or WO2005/080001 (in particular also Fig. 1 and 2).

The liquid 2 may contain or consist of pharmacologically active substances or mixtures of substances, preferably selected from those groups:

### Anticholinergica:

Anticholinergica preferably selected from the group consisting of tiotropium, tiotropiumbromide, oxitropiumbromide, flutropiumbromide, ipratropiumbromide, glycopyrroniumsalts, trospiumchloride, tolterodin, 2,2-diphenylpropionacidtropenolester-methobromide, 2,2-diphenylpropionacidscopinester-methobromide, 2-fluoro-2,2-diphenylacidicacidscopinester-methobromide, 2-fluoro-2,2-diphenylacidicacidtropenolester-methobromide, 3,3',4,4'-tetrafluorbenzilacidtropenolester-methobromide, 3,3,4,4'-tetrafluorbenzilacidscopinestermethobromide, 4,4'-difluorbenzilacidtropenolester-methobromide, 4,4'-difluorbenzilacidscopinester-methobromide, 3,3'-difluorobenzilacidtropenolestermethobromide, 3,3'-diflaorobenzilacidscopinester-methobromide, 9-hydroxyfluoren-9-carbonacidtropenolester -methobromide, 9-fluoro-fluoren-9-carbonacidtropenolester-methobromide, 9-hydroxy-fluoren-9-carbonacidscopinester-methobromide, 9-fluoro-fluoren-9-carbonacidscopinester-methobromide, 9-methyl-fluoren-9-carbonacidtropenoleste- methobromide, 9-methylfluoren-9-carbonacidscopineste- methobromide, benzilacidcyclopropyltropinester-methobromide, 2,2-diphenylpropionacidcyclopropyltropinester -methobromide, 9-hydroxy-xanthen-9-carbonacidcyclopropyltropinester-methobromide, 9-methyl-fluoren-9-carbonacidcyclopropyltropinester-methobromide, 9-methyl-xanthen-9-carbonacidcyclopropyltropinester-methobromide, 9-hydroxy-fluoren-9-carbonacidcyclopropyltropinester-methobromide, 4,4'-difluorbenzilacidmethylestercyclopropyltropinester-methobromide, 9-hydroxy-xanthen-9-carbonacidtropenolester-methobromide, 9-hydroxy-xanthen-9-carbonacidscopinester-methobromide, 9-methyl-xanthen-9-carbonacidtropenolestermethobromide, 9-methyl-xanthen-9-carbonacidscopinester-methobromide, 9-ethyl-xanthen-9-carbonacidtropenolester-methobramide, 9-difluormethylxanthen-9-carbonacidtropenolester-methobromide, 9-hydroxymethyl-xanthen-9-carbonacidscopinester-methobromide, optionally in the form of the racemates, the enantiomers, the diastereomers and optionally the pharmacologically acceptable acid addition salts and the hydrates thereof

### Beta-sympathomimetica:

Beta-sympathomimetica preferably selected from the group consisting of albuterol, bambuterol, bitolterol, broxaterol, carbuterol, clenbuterol, fenoterol, formoterol, hexoprenaline, ibuterol, indacterol, isoetharine, isoprenaline, levosalbutamol, mabuterol, meluadrine, metaproterenol, orciprenaline, pirbuterol, procaterol, reproterol, rimiterol, ritodrine, salmeterol, salmefamol, soterenot, sulphonterol, tiaramide, terbutaline, tolubuterol, CHF-1035, HOKU-81, KUL-1248, 3-(4-{6-[2-hydroxy-2-(4-hydroxy-3-hydroxymethyl-phenyl)-ethylamino]-hexyloxy}-butyl)-benzenesulfoneamide, 5-[2-(5,6-Diethyl-indan-2-ylamino)-1-hydroxy-ethyl]-8-hydroxy-1*H*-quinolin-2-one, 4-hydroxy-7-[2-{[2-{[3-(2-phenylethoxy)propyl]sulphonyl}ethyl]-ammo}ethyl]-2 (3H)-benzothiazolone, 1-(2-fluoro-4-hydroxyphenyl)-2-[4-(1-benzimidazolyl)-2-methyl-2-butylamino]ethanol, 1-[3-(4-methoxybenzyl-amino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-methoxyphenyl)-2-methyl-2-propylamino]ethanol, 1-[2H-5-hydroxy-3-oxo-4H-1,4-benzoxazin-8-yl]-2-[3-(4-n-butyloxyphenyl)-2-methyl-2-propylamino]ethanol, 1-[2H-5-hydroxy-3-oxo-4H-1,4-benzoxazin-8-yl]-2-{4-[3-(4-methoxyphenyl)-1,2,4-triazol-3-yl]-2-methyl-2-butylammo}ethanol, 5-hydmxy-8-(1-hydroxy-2-isopropylaminobutyl)-2H-1,4-benzoxazin-3-(4H)-one, 1-(4-amino-3-chloro-5-trifluormethylphenyl)-2-tert.-butylamino)ethanol und 1-(4-ethoxycarbonylamino-3-cyano-5-fluorophenyl)-2-(tert.-butylamino)ethanol, optionally in the form of the racemates, the enantiomers, the diastereomers and optionally the pharmacologically acceptable acid addition salts and the hydrates thereof.

### Steroids:

Steroids preferably selected from the group consisting of prednisolone, prednisone, butixocortpropionate, RPR-106541, flunisolide, beclomethasone, triamcinolone, budesonide, fluticasone, mometasone, ciclesonide, rofleponide, ST-126, dexamethasone, 6α,9α-difluoro-17α-[(2-furanylcarbonyl)oxy]-11β-hydroxy-16α-methyl-3-oxo-androsta-1,4-dien-17β-carbothionacid (S)-fluoromethylester, 6α,9α-difluoro-11β-hydroxy-16α-methyl-3-oxo-17α-propionyloxy-androsta-1,4-dien-17β-carbothionacid (S)-(2-oxo-tetrahydro-furan-3S-yl) ester and etiprednol-dichloroacetat (BNP-166), optionally in the form of the racemates, the enantiomers, the diastereomers and optionally the pharmacologically acceptable acid addition salts and the hydrates thereof.

### PDEIV-inhibitors:

PDE IV-inhibitor preferably selected from the group consisting of enprofyllin, theophyllin, roflumilast, ariflo (cilomilast), CP-325,366, BY343, D-4396 (Sch-351591), AWD-12-281 (GW-842470), N-(3,5-Dichloro-l-oxo-pyridin-4-yl)-4-difluoromethoxy-3-cyclopropylmethoxybenzamide, NCS-613, pumafentine, (-)p-[(4*a*R*,10*b*S*)-9-ethoxy-1,2,3,4,4a,10b-hexahydro-8-methoxy-2-methylbenzo[s][1,6]naphthyridin-6-yl]-N,N-diisopropylbenzamide, (R)-(+)-1-(4-bromobenzyl)-4-[(3-cyclopentyloxy)-4-methoxyphenyl]-2-pyrrolidone, 3-(cyclopentyloxy-4-methoxyphenyl)-1-(4-N-[N-2-cyano-S-methyl-isothioureido]benzyl)-2-pyrrolidone, cis[4-cyano-4-(3-cyclopentyloxy-4-methoxyphenyl)cyclohexan-1-carbonacid], 2-carbomethoxy-4-cyano-4-(3-cyclopropylmethoxy-4-difluoromethoxyphenyl)cyclohexane-1-on, cis[4-cyano-4-(3-cyclopropylmethoxy-4-difluoromethoxyphenyl)cyclohexane-1-ol], (R)-(+)-ethyl[4-(3-cyclopentyloxy-4-methoxyphenyl)pyrrolidin-2-yliden]acetate, (S)-(-)-ethyl[4-(3-cyclopentyloxy-4-methoxyphenyl)pyrrolidin-2-yliden]acetate, CDP840, Bay-198004, D-4418, PD-168787, T-440, T-2585, arofyllin, atizoram, V-11294A, Cl-1018, CDC-801, CDC-3052, D-22888, YM-58997, Z-15370, 9-cyclopentyl-5,6-dihydro-7-ethyl-3-(2-thienyl)-9*H*-pyrazolo[3,4-c]-1,2,4-triazolo[4,3-a]pyridin and 9-cyclopentyl-5,6-dihydro-7-ethyl-3-(*tert-*butyl)-9*H*-pyrazolo[3,4-c]-1,2,4-triazolo[4,3-a]pyridine, optionally in the form of the racemates, the enantiomers, the diastereomers and optionally the pharmacologically acceptable acid addition salts and the hydrates thereof.

### LTD4-Antagonists:

LTD4-antagonist preferably selected from the group consisting of montelukast, 1-(((R)-(3-(2-(6,7-difluoro-2-quinolinyl)ethenyl)phenyl)-3-(2-(2- hydroxy-2-propyl)phenyl)thio)methylcyclopropan-acidicacid, 1-(((1(R)-3(3-(2-(2,3-dichlorothieno[3,2-b]pyridin-5-yl)-(E)-ethenyl)phenyl)-3-(2-(1-hydroxy-1-methylethyl)phenyl)propyl)thio)methyl)cyclopropanacidicacid, pranlukast, zafirlukast, [2-[[2-(4-tert-butyl-2-thiazolyl)-5-benzofuranyl]-oxymethyl]phenyl]acidicacid, MCC-847 (ZD-3523), MN-001, MEN-91507 (LM-1507), VUF-5078, VUF-K-8707 und L-733321, optionally in the form of the racemates, the enantiomers, the diastereomers and optionally the pharmacologically acceptable acid addition salts and the hydrates thereof.

### EGFR-Kinase-Inhibitors:

cetuximab, trastuzumab, ABX-EGF, Mab ICR-62, 4-[(3-Chlor-4-fluorophenyl)amino]-6-{[4-(morpholin-4-yl)-1-oxo-2-buten-1-yl]-amino}-7-cyclopropylmethoxy-chinazolin, 4-[(R)-(1-phenyl-ethyl)amino]-6-{[4-(morpholin-4-yl)-1-oxo-2-buten-1-yl]-amino}-7-cyclopentyloxy-chinazolin, 4-[(3-chloro-4-fluoro-phenyl)amino]-6-{[4-((R)-6-methyl-2-oxo-morpholin-4-yl)-1-oxo-2-buten-1-yl]amino}-7-[(S)-(tetrahydrofurane-3-yl)oxy]-chinazoline, 4-[(3-chloro-4-fluoro-phenyl)amino]-6-[2-((S)-6-methyl-2-oxo-morpholine-4-yl)-ethoxy]-7-methoxy-chinazolin, 4-[(3-chloro-4-fluorophenyl)amino)-6-({4-[N-(2-methoxy-ethyl)-N-methyl-amino]-1-oxo-2-buten-1-yl}amino)-7-cyclopropylmethoxy-chinazoline, 4-[(R)-(1-Phenyl-ethyl)amino]-6-({4-[N-(tetrahydropyrane-4-yl)-N-methyl-amino]-1-oxo-2-buten-1-yl}amino)-7-cyclopropylmethoxy-chinazoline, 4-[(3-chloro-4-fluorophenyl)amino]-6-({4-[N-(2-methoxy-ethyl)-N-methyl-amino]-1-oxo-2-buten-1-yl}ammo)-7-cyclopentyloxychinazoline, 4-[(3-chloro-4-fluorophenyl)amino]-6-{[4-(N,N-dimethylamino)-1-oxo-2-buten-1-yl]amino}-7-[(R)-(tetrahydrofuran-2-yl)methoxy]-chinazolin, 4-[(3-Ethinyl-phenyl)amino]-6,7-bis-(2-methoxy-ethoxy)-chinazoline, 4-[(R)-(1-phenyl-ethyl)amino]-6-(4-hydroxy-phenyl)-7H-pyrrolo[2,3-d]pyrimidine, 3-cyano-4-[(3-chloro-4-fluorophenyl)amino]-6-{[4-(N,N-dimethylamino)-1-oxo-2-buten-1-yl]amino}-7-ethoxy-chinoline, 4-[(R)-(1-phenylethyl)amino]-6-{[4-((R)-6-methyl-2-oxo-morpholin-4-yl)-1-oxo-2-buten-1yl]-amino}-7-methoxy-chinazoline, 4-[(3-chloro-4-fluorophenyl)amino]-6-{[4-(morpholine-4-yl)-1-oxo-2-buten-1-yl]-amino}-7-[(tetrahydrofurane-2-yl)-methoxy]-chinazoline, 4-[(3-ethinyl-phenyl)amino]-6-{[4-(5.5-dimethyl-2-oxo-mor-pholin-4-yl)-1-oxo-2-buten-1-yl]amino}-chinazoline, 4-[(3-chloro-4-fluoro-phenyl)ammo]-6-{2-[4-(2-oxo-morpholine-4-yl)-piperidine-1-yl]-ethoxy}-7-methoxy-chinazoline, 4-[(3-chloro-4-fluoro-phenyl)amino]-6-(trans-4-amino-cyclohexan-1-yloxy)-7-methoxy-chinazoline, 4-[(3-chloro-4-fluoro-phenyl)amino]-6-(trans-4-methansulfonylamino-cyclohexan-1-yloxy)-7-methoxy-chinazoline, 4-[(3-chloro-4-fluorc-phenyl)amino]-6-(tetrahydropyrane-3-yloxy)-7-methoxy-chinazoline, 4-[(3-chloro-4-fluoro-phenyl)amino]-6-{1-[(morpholine-4-yl)carbonyl]-piperidin-4-yl-oxy}-7-methoxy-chinazoline, 4-[(3-chloro-4-fluoro-phenyl)amino]-6-{piperidine-3-yloxy)-7-methoxy-chinazoline, 4-[(3-chloro-4-fluoro-phenyl)amino]-6-[1-(2-acetylamino-ethyl)-piperidin-4-yloxy]-7-methoxy-chinazoline, 4-[(3-chloro-4-fluoro-phenyl)-amino]-6-(tetrahydropyran-4-yloxy)-7-ethoxy-chinazoline, 4-[(3-chloro-4-fluoro-phenyl)aminol-6-{trans-4-[(morpholin-4-yl)carbonylamino]-cyclohexan-1-yloxy}-7-methoxy-chinazoline, 4-[(3-chloro-4-fluoro-phenyl)amino]-6-{1-[(piperidin-1-yl)carbonyl]-piperidin-4-yloxy}-7-methoxy-chinazoline, 4-[(3-chloro-4-fluoro-phenyl)amino]-6-(cis-4-{N-[(morpholin-4-yl)carbonyl]-N-methyl-amino}-cyclohexan-1-yloxy)-7-methoxy-chinazoline, 4-[(3-chloro-4-fluoro-phenyl)amino]-6-(trans-4-ethansulfonylamino-cyclohexan-1-yloxy)-7-methoxy-chinazoline, 4-[(3-chloro-4-fluoro-phenyl)amino]-6-(1-methansulfonyl-piperidin-4-yloxy)-7-(2-methoxy-ethoxy)-chinazoline, 4-[(3-chloro-4-fluoro-phenyl)amino]-6-[1-(2-methoxy-acetyl)-piperidin-4-yloxy]-7-(2-methoxy-ethoxy)-chinazoline, 4-[(3-ethinyl-phenyl)amino]-6-(tetrahydropyran-4-yloxy]-7-methoxy-chinazoline, 4-[(3-chloro-4-fluoro-phenyl)amino]-6-(cis-4-{N-[(piperidin-1-yl)carbonyl]-N-methyl-amino}-cyclohexan-1-yloxy)-7-methoxy-chinazoline, 4-[(3-chloro-4-fluoro-phenyl)amino]-6-{cis-4-[(morpholin-4-yl)carbonylamino]-cyclohexan-1-yloxy}-7-methoxy-chinazoline, 4-[(3-chloro-4-fluoro-phenyl)amino]-6-{1-[2-(2-oxopyrrolidin-1-yl)-ethyl]-piperidin-4-yloxy}-7-methoxy-chinazoline, 4-[(3-ethinyl-phenyl)amino]-6-(1-acetyl-piperidin-4-yloxy)-7-methoxy-chinazoline, 4-[(3-ethinylphenyl)amino]-6-(1-methyl-piperidin-4-yloxy)-7-methoxy-chinazoline, 4-[(3-ethinyl-phenyl)amino]-6-(1-methansulfonyl-piperidin-4-yloxy)-7-methoxy-chinazolin, 4-[(3-chloro-4-fluoro-phenyl)amino]-6-(1-methyl-piperidin-4-yloxy)-7(2-methoxy-ethoxy)-chinazoline, 4-[(3-ethinyl-phenyl)amino]-6-{1-[(morpholin-4-yl)carbonyl]-pipendin-4-yloxy}-7-methoxy-chinazoline, 4-[(3-chloro-4-fluoro-phenyl)amino]-6-{1-[(N-methyl-N-2-methoxyethyl-amino)-carbonyl]-piperidin-4-yloxy}-7-methoxy-chinazoline, 4-[(3-chloro-4-fluoro-phenyl)amino]-6-(1-ethyl-piperidin-4-yloxy)-7-methoxy-chinazoline, 4-[(3-chloro-4-fluoro-pbenyl)aminol-6-[cis-4-(N-methansulfonyl-N-methyl-amino)-cyclohexan-1-yloxy]-7-methoxy-chinazoline, 4-[(3-chloro-4-fluoro-phenyl)-amino]-6-[cis-4-(N-acetyl-N-methyl-amino)-cyclohexan-1-yloxy]-7-methoxy-chinazoline, 4-[(3-chloro-4-fluoro-phenyl)amino]-6-(trans-4-methylamino-cyclohexan-1-yloxy)-7-metboxy-chinazoline, 4-[(3-chloro-4-fluoro-phenyl)-amino]-6-[trans-4-(N-methansulfonyl-N-methyl-amino)-cyclohexan-1-yloxy]-7-methoxy-chinazoline, 4-[(3-chloro-4-fluoro-phenyl)amino]-6-(trans-4-dimethylamino-cyclohexan-1-yloxy)-7-methoxy-chinazoline, 4-[(3-chloro-4-fluoro-phenyl)amino]-6-(trans-4-{N-[(morpholin-4-yl)carbonyl]-N-methylamino}-cyclohexan-1-yloxy)-7-methoxy-chinazoline, 4-[(3-chloro-4-fluorophenyl)amino]-6-[2-(2,2-dimethyl-6-oxo-morpholin-4-yl)-ethoxy]-7-[(S)-(tetrahydrofuran-2-yl)methoxy]-chinazoline, 4-[(3-chloro-4-fluoro-phenyl)-amino]-6-(1-methansulfonyl-piperidin-4-yloxy)-7-methoxy-chinazoline, 4-[(3-chlor-4-fluoro-phenyl)amino]-6-(1-cyano-piperidin-4-yloxy)-7-methoxy-chinazoline, und 4-[(3-chloro-4-fluoro-phenyl)amino]-6-{1-[(2-methoxyethyl)carbonyl]-piperidin-4-yloxy}-7-methoxy-chinazoline, optionally in the form of the racemates, the enantiomers, the diastereomers and optionally the pharmacologically acceptable acid addition salts and the hydrates thereof.

Moreover, the compound could be from the group of betamimetika, antiallergika, derivates of ergotalcaloids, triptane, CGRP-antagonists, phosphodiesterase-V-inhibitores, optionally in the form of the racemates, the enantiomers, the diastereomers and optionally the pharmacologically acceptable acid addition salts and the hydrates therof.

The pharmacologically acceptable acid addition salts could be from the group of hydrochloride, hydrobromide, hydroiodide, hydrosulfate, hydrophosphate, hydromethansulfonate, hydronitrate, hydromaleate, hydroacetate, hydrobenzoate, hydrocitrate, hydrofumarate, hydrotartrate, hydrooxalate, hydrosuccinate, hydrobenzoate und hydro-p-toluolsulfonate, preferably hydrochloride, hydrobromide, hydrosulfate, hydrophosphate, hydrofumarate and hydromethansulfonate.

As antiallergika: disodiumcromoglicate, nedocromil.

As derivates of alkaloides: dihydroergotamine, ergotamine.

Moreover, inhalable macromolecules can be used as pharmacologically active substances, as disclosed in EP 1 003 478.

For inhalation purposes pharmaceuticals, formulations and mixtures of pharmaceuticals with the above named pharmacologically active substances can be used, as well as their pharmacologically active salts, esters and combinations of the pharmacologically active substances, salts and esters.

## Claims

1. Dispensing device (1) for dispensing a liquid (2), in particular containing or consisting of a drug, as a fine spray (3), the dispensing device (1) comprising a nozzle (4) having a duct (5) or slit (22) through which the liquid (2) is dispensable, the duct (5) or slit (22) having a flat cross section, the ratio of the largest side (d1) to the smallest side (d2) of the flat cross section being at least 2.0.

2. Dispensing device according to claim 1, **characterized in that** the ratio is between 2 to 20, preferably about 3 to 10.

3. Dispensing device according to claim 2, **characterized in that** the largest side (d1) is between 0.5 to 5 mm, preferably about 1 to 3 mm.

4. Dispensing device according to any one of the preceding claims, **characterized in that** the smallest side (d2) is between 0.05 to 0.3 mm, preferably about 0.07 to 0.2 mm.

5. Dispensing device according to any one of the preceding claims, **characterized in that** the flat cross section is substantially oval or rectangular.

6. Dispensing device (1) for dispensing a liquid (2), in particular containing or consisting of a drug, as a fine spray (3), preferably according to any one of the preceding claims, the dispensing device (1) comprising a nozzle (4) having a duct (5) through which the liquid (2) is dispensable, the nozzle (4) or duct (5) comprising at least one sharp change (12) in cross section and/or alternate sections (13,14) with large and small cross sectional areas.

7. Dispensing device according to claim 6, **characterized in that** the change (12) is step-like.

8. Dispensing device according to claim 6 or 7, **characterized in that** the change (12) is more than 20%, preferably more than 50 %, in cross sectional area.

9. Dispensing device according to any one of claims 6 to 8, **characterized in that** the change (12) is formed by a surface extending at least substantially radially or transversely to the main flow direction of the liquid (2).

10. Dispensing device according to any one of claims 6 to 9, **characterized in that** transitions between the alternate sections (13, 14) form sharp or step-like changes (12) in cross section.

11. Dispensing device according to any one of claims 6 to 10, **characterized in that** the alternate sections (13) have similar cross sectional shapes.

12. Dispensing device according to any one of claims 6 to 11, **characterized in that** the ratio of the cross sectional area of the large section(s) (13) to the cross sectional area of the small section(s) (14) is more than 1.2, preferably more than 1.5.

13. Dispensing device according to any one of claims 6 to 12, **characterized in that** the alternate sections (13, 14) respectively have a length of 1 to 5 mm or more.

14. Dispensing device according to any one of claims 6 to 13, **characterized in that** the cross section of the duct (5) is substantially flat, oval, rectangular or square.

15. Dispensing device according to any one of the preceding claims, **characterized in that** the cross sectional area of the duct (5) is between 0.02 and 0.4 mm².

16. Dispensing device according to any one of the preceding claims, **characterized in that** the mean hydraulic diameter of the duct (5) is less than 1 mm, preferably about 0.1 to 0.6 mm.

17. Dispensing device according to any one of the preceding claims, **characterized in that** the ratio of the length of the duct (5) to the mean hydraulic diameter of the duct (5) is between 5 and 1000, preferably between 10 and 250.

18. Dispensing device according to any one of the preceding claims, **characterized in that** the nozzle (4) comprises multiple ducts (5) in parallel.

19. Dispensing device according to any one of the preceding claims, **characterized in that** the dispensing device (1) comprises a means for slowing down the propagation velocity of the spray (3), in particular a diffuser (15), preferably at the exit of the nozzle (4) or duct (5).

20. Dispensing device according to any one of the preceding claims, **characterized in that** the dispensing device (1) comprises a jet impinging means (17) for impinging at least two jets (P) to slow down the propagation velocity of the spray (3) and/or to mix separate drugs or liquids (2).

21. Dispensing device according to any one of the preceding claims, **characterized in that** the dispensing device (1) comprises compressed or liquefied gas for dispensing the liquid (2).

22. Dispensing device according to claim 21, **characterized in that** the dispensing device (1) comprises a reservoir (8) containing the liquid (2) and the gas.

23. Dispensing device according to any one of the preceding claims, **characterized in that** the liquid (2) contains liquefied gas and/or at least one liquid drug.

24. Dispensing device according to any one of the preceding claims, **characterized in that** the liquid (2) is a solution, suspension or dispersion.

25. Dispensing device according to any one of the preceding claims, **characterized in that** the dispensing device (1) comprises a metering device, in particular a metering valve (9), for metering the amount of liquid (2) dispensed during each dispensing operation.

26. Dispensing device according to any one of the preceding claims, **characterized in that** the dispensing device (1) comprises a monitoring device (19) for counting the number of actuated dispensing operations or remaining dispensing operations.

27. Dispensing device according to any one of the preceding claims, **characterized in that** the dispensing device (1) is a preferably oral inhaler, in particular a metering dose inhaler.

28. Dispensing device according to any one of the preceding claims, **characterized in that** the dispensing device (1) comprises the liquid (2) containing at least one of an anticholinergicum, a beta-sympathomimeticum, a steroid, a PDEIV-inhibitor, a LTD4-antagonist, an EGFR-kinase-inhibitor, and antiallergicum.
